# EUROPEAN PATENT SPECIFICATION

(11) **EP 3 493 744 B1**
(45) Date of publication and mention of the grant of the patent: **27.11.2019**
(21) Application number: 17751051.8
(22) Date of filing: 28.07.2017
(51) Int. Cl.: A61B 8/06, A61B 8/08, A61B 8/12, A61B 8/00, A61B 8/04

(54) **SYSTEM FOR DETERMINING CARDIAC OUTPUT**
SYSTEM ZUR BESTIMMUNG DER HERZLEISTUNG
SYSTÈME PERMETTANT DE DÉTERMINER LE DÉBIT CARDIAQUE

(30) Priority: 02.08.2016 EP 16182365; 02.12.2016 EP 16201941
(43) Date of publication of application: 12.06.2019
(73) Proprietor: Koninklijke Philips N.V., 5656 AE Eindhoven (NL)
(72) Inventor: VAN DER HORST, Arjen, 5656 AE Eindhoven (NL); SIO, Charles, Frederik, 5656 AE Eindhoven (NL); KUENEN, Maarten, Petrus, Joseph, 5656 AE Eindhoven (NL)
(74) Representative: Philips Intellectual Property & Standards
(86) International application number: PCT/EP2017/069103
(87) International publication number: WO 2018/024618

(56) References cited:
- EP-A2- 0 503 839
- WO-A1-2013/122459
- US-A1- 2008 051 661
- US-A1- 2016 000 403
- US-A1- 2016 007 953

## Description

### FIELD OF THE INVENTION

The invention relates to a system and software product for determining a representation of cardiac output in a patient.

### BACKGROUND OF THE INVENTION

Cardiac output (CO) measurement is an important tool in monitoring the hemodynamic status of patients during surgery and during their stay on the intensive care unit (ICU). Cardiac output is the volume of blood pumped by the heart in a given time. As the blood circulation is a closed system, this can mean either the volume of blood pumped into the aorta by the left ventricle, but equally the volume of blood pumped into the pulmonary artery by the right ventricle, or the volume of blood flowing into the right atrium (venous return) or left atrium.

Extracorporeal ultrasound cannot determine CO accurately enough. Intravascular measurements are called for, but there are severe risks involved with prolonged intravascular blood flow measurements in the aorta. The Pulmonary Artery Catheter (PAC) or Swan-Ganz catheter is still the gold standard for CO measurements. The PAC is inserted in the venous system and is advanced via the right atrium and ventricle into the pulmonary artery, which is not without risk. The PAC enables CO measurement via thermodilution methods. However, its use has been associated with various complications during and after the measurement procedure. This has led to development of several minimally invasive methods of CO monitoring based on (combinations of) thermodilution, transthoracic or - esophageal (Doppler) ultrasound and pressure pulse power or contour analysis. However, these methods all have their drawbacks, either in practicality (limited frequency of measuring, hands-on operation, time needed for a measurement and/or patient discomfort) or accuracy in a large range of hemodynamic conditions.

Recently, new devices have been proposed and/or introduced to measure the flow velocity in the Superior Vena Cava (SVC) using a US Doppler sensor equipped Central Venous Catheter (CVC) and Peripheral Inserted Central Catheters (PICC) for fluid responsiveness monitoring and navigation. These devices could potentially enable CO monitoring without the complications related to the PAC.

Normally, the flow in the Superior Vena Cava accounts for 35% of the total venous return and thus CO. About 60% of the total venous return goes via the Inferior Vena Cava (IVC) and 5% drains directly in the right atrium from the coronary circulation, mainly via the Coronary Sinus (CS). The ratio of flow in the Superior Vena Cava and Inferior Vena Cava varies between patients and may be influenced by disease, but is in most cases quite stable. However, hemodynamic instability (e.g. during surgery, or in ICU patients) may cause these ratios to change rapidly over time. This means that measuring the flow in the Superior Vena Cava alone cannot serve as a surrogate for CO.

United States Patent Application US2016/000403 describes a method of measuring the cardiac output. The method uses an ultrasound emitter and one or more receivers placed in the superior vena cava just above the right atrium of the heart so that the ultrasound apparatus can transmit through the wall of the superior vena cava and the juxtaposed wall of the aorta at this location. By measuring the velocity of the blood by its back-scattered Doppler shift, the cardiac output can be determined.

### SUMMARY OF THE INVENTION

It is an object of the present invention to provide an improved system and software product for determining a representation of cardiac output in a patient that mitigates the problems of the known systems and approaches.

In a first aspect of the present invention a system for determining a representation of cardiac output in a patient is presented comprising a transducer unit for transmitting to and receiving ultrasound signals from the Superior Vena Cava or the Inferior Vena Cava of the patient, and a processor arranged to control the transducer unit, wherein the controlling includes controlling of a first and a second timing of receiving ultrasound signals in relation to a transmission of ultrasound signals, and to process the ultrasound signals received at the first and the second timing, so determine a first and a second velocity of blood, the first velocity of blood being that of blood in the Superior Vena Cava and the second velocity of blood being that of blood in the Inferior Vena Cava, wherein the processor is further arranged to determine the representation of cardiac output from the first and the second velocity.

The system of the present invention enables a method for determining a representation of cardiac output in a patient, the method comprising causing a transducer unit positioned in the Superior Vena Cava or the Inferior Vena Cava of the patient to transmit and to receive ultrasound signals, wherein the receiving of ultrasound signals is provided at a first and a second timing of in relation to a transmission of ultrasound signals, processing the ultrasound signals received at the first and the second timing, so determine a first and a second velocity of blood, the first velocity of blood being that of blood in the Superior Vena Cava and the second velocity of blood being that of blood in the Inferior Vena Cava, and determining a representation of a cardiac output from the first and the second velocity.

According to the present invention problems of the known systems and approaches are mitigated by using a single device in the Superior Vena Cava (or Inferior Vena Cava) that can determine both the velocity of blood in the Superior Vena Cava and Inferior Vena Cava. This enables continuous monitoring of cardiac output without the complications related to the PAC.

It was realized by the inventors that, while just the information on the flow velocity in the Superior Vena Cava (or the Inferior Vena Cava) alone is - in practice - not sufficient for reliably deducing the cardiac output of the patient, the use of the same ultrasound transducer unit (possibly including multiple transducer elements) for determining the blood velocity at two different locations (one in the Superior Vena Cava and the other one in the Inferior Vena Cava) allows for determining a close representation of the cardiac output, which basically corresponds to the combined flow through the Superior Vena Cava and the Inferior Vena Cava, besides a minor portion of blood from the coronary circulation.

In an embodiment of the present invention a system for ultrasound based monitoring of cardiac output in a patient is provided, wherein the system comprises: a connection for connecting to an ultrasound transducer arranged to be positioned in a patient for transmitting and receiving ultrasound signals in order to determine a velocity of a fluid at a distance from the ultrasound transducer, and a processor arranged: to control the ultrasound transducer to transmit at a start time (t0) ultrasound signals, to control the ultrasound transducer to receive at a first time (t0 + Δt1), which is after the start time, ultrasound signals and to determine a first velocity of fluid at a first distance from the ultrasound transducer, to control the ultrasound transducer to receive at a second time (t0 + Δt2), which is after the first time, ultrasound signals and to determine a second velocity of fluid at a second distance from the ultrasound transducer, and to combine the first velocity and the second velocity as a representation of the cardiac output.

In a further embodiment of the system according to the present invention a method of ultrasound based monitoring of cardiac output in a patient is enabled, the method comprising: positioning an ultrasound transducer in the Superior Vena Cava of a patient for transmitting and receiving ultrasound signals in order to determine a velocity of a blood at a distance from the ultrasound transducer, controlling the ultrasound transducer to transmit at a start time (t0) ultrasound signals, controlling the ultrasound transducer to receive at a first time (t0 + Δt1), which is after the start time, ultrasound signals and determining the velocity of blood in the Superior Vena Cava, controlling the ultrasound transducer to receive at a second time (t0 + Δt2), which is after the first time, ultrasound signals and to determine the velocity of blood in the Inferior Vena Cava, and combining the velocity of blood in the Superior Vena Cava and the velocity of blood in the Inferior Vena Cava as a representation of the cardiac output.

In a preferred embodiment, the processor is further arranged to determine the first and second timing based on distance information inputted to the system, the distance information indicating a distance between the transducer unit and a region of interest in the Superior Vena Cava and between the transducer unit and a region of interest in the Inferior Vena Cava, and/or to determine the first and second timing based on a velocity profile along a line of sight of the transducer unit.

The timing of transmission and reception of ultrasound signals is related to the distance between the transducer and the region of interest and the propagation velocity of the ultrasound inside the body of the patient (i.e. in blood and/or tissue). From images of the relevant anatomy of the patient and in view of knowledge of the position of the transducer in the patient's body, distance information may be obtained and inputted into the system. Alternatively, the system may determine the timing using the opposite flow directions of the blood in the Superior Vena Cava and the Inferior Vena Cava, respectively, upon determining velocities of the blood along a line of sight. These approaches may be combined, for example in the form of using inputted distance information for defining positional ranges for determination of the blood velocity in the Superior Vena Cava and the Inferior Vena Cava, which are then confirmed by means of the velocity profile.

In a preferred embodiment, the processor is further arranged to control the transducer unit in an imaging mode, and the processor is further arranged to derive anatomical information from an ultrasound image and to determine the first and second timing based on the derived anatomical information; and/or to derive anatomical information from an ultrasound image, wherein the anatomical information includes a diameter of the Superior Vena Cava and/or the Inferior Vena Cava, wherein the diameter is used in determining the representation of the cardiac output.

In addition or in alternative to the above embodiment, the ultrasound transducer of this embodiment provides image data, which is used by the system to determine the timing based on the situation and location of the transducer in situ and/or to obtain further information, i.e. the diameter of the blood vessels in question, which in combination with the velocities is of interest in the context of determining the cardiac output. The provision of the imaging mode may be intermittent to the provision of the mode of determining the flow velocities, while it is also possible that the imaging mode is used only, e.g. once, at the beginning of the procedure. In case of multiple transducer units included in the transducer, the different modes may be provided simultaneously or in an overlapping way.

In a preferred modification of the above embodiment, the transducer unit includes an intravascular ultrasound transducer, in particular an additional intravascular ultrasound transducer in the form of an IVUS transducer (preferably looking sideways) for determining the diameter of the vessel.

The provision of intravascular ultrasound with a sufficiently low IVUS frequency allows a penetration depth for visualization of the vessel walls. It is possible to use a rotational and/or a phased array IVUS configuration.

In a preferred embodiment, the processor is arranged to control the transducer unit so to provide a series of transmission and receptions, and the processor is arranged to determine the first and the second velocity from an ensemble of received ultrasound signals corresponding to the series of receptions.

Among other implementations (e.g. a Fourier analysis), it is possible to determine the flow velocities in the Superior Vena Cava and the Inferior Vena Cava by analysis of the ensemble of signals by means of a pulsed Doppler velocity estimation algorithm, as it is described, for example, in Kasai C. et al, "Real-Time Two-Dimensional Blood Flow Imaging Using an Autocorrelation Technique" (IEEE Transactions on Sonics and Ultrasonics, Vol. 32(3), pages 458 to 464, 1985).

In a preferred modification of the above embodiment, the representation of cardiac output is determined by averaging over one or more heart cycles.

The averaging allows for a more smooth presentation of the information on the cardiac output.

In a preferred embodiment, the processor is arranged to control the transducer unit such that a first and a second ultrasound signal is transmitted, wherein the first timing of receiving is in relation to the transmission of the first ultrasound signal and the second timing of receiving is in relation to the transmission of the second ultrasound signal.

The first and second transmitted ultrasound signal may, in particular, differ in nature, e.g. as to at least one or more of center frequency, pulse length, beam steering, amplitude, etc.

With different signals used for determining the flow velocities in the Superior Vena Cava and the Inferior Vena Cava it is possible to optimize the transmission signals for the respective blood vessel, in particular in terms of center frequency and/or pulse length.

In a preferred embodiment, the transducer unit includes a first transducer element and a second transducer element, wherein the processor is arranged to independently control the first and the second transducer element, wherein the first timing of receiving is in relation to a transmission of an ultrasound signal by the first transducer element and the second timing of receiving is in relation to a transmission of an ultrasound signal by the second transducer element.

The first and second transmitted ultrasound signal may, in particular, differ in nature, e.g. as to at least one or more of center frequency, pulse length, beam steering, amplitude, etc. The different transducer elements also allow for further optimization beyond or besides the use of different signals, for example in the provision of acoustic lenses, each adapted for a certain distance (range), while other measures known to the skilled person may be used for further improving, for example, the signal to noise ratio. With the transducer elements using non-overlapping frequency bands, the different velocities in the Superior Vena Cava and the Inferior Vena Cava can be determined at the same time without crosstalk or interference.

In a preferred embodiment, the transducer unit is provided such that the orientation of the transducer unit, a transmitted beam and/or a received beam is adjustable and the processor is further arranged to adjust an orientation of the transducer unit, the transmitted beam and/or the received beam so to provide a first orientation at the first timing of receiving and a second orientation at the second timing of receiving.

Depending on the nature of the transducer unit, it might be difficult to reorient the transducer unit in the time frame from to first timing to the second timing, so the embodiment includes also the case that there is a transmission and receiving under the first orientation and a (further) transmission and receiving under the second orientation.

Even though the Superior Vena Cava and the Inferior Vena Cava are typically aligned to a certain degree and may therefore be monitored by means of a transducer having a single line of sight or transducer axis, further optimization of the determination of the representation of the cardiac output may be achieved by introducing some degree of flexibility as to the direction of the ultrasound emission and reception, for instance by beam steering or beam forming.

In a preferred embodiment, the system further comprises a pressure sensor for detecting a pressure in the Superior Vena Cava or the Inferior Vena Cava of the patient, wherein the processor is arranged to use the detected pressure in the determination of the representation of cardiac output.

It is possible to measure, for example, the central venous pressure. This information may be used in the determination of the representation of the cardiac output, in particular as the diameter of the blood vessels varies with the blood pressure. The pressure sensor can be provided together with the ultrasound transducer. It is also possible to detect the pressure via a lumen of a CVC or PICC with an external pressure transducer.

In a preferred embodiment, the processor is further arranged to monitor a change in a ratio between the first and the second velocity over time and to provide a notification in case the change exceeds a predetermined threshold.

Typically, the Superior Vena Cava accounts for about 35 % of the total venous return, while about 60% of the total venous return passes through the Inferior Vena Cava. The remaining (about) 5% of the total venous return drain directly from the coronary circulation into the right atrium, mainly via the Coronary Sinus (CS). The exact values vary from patient to patient, while for each patient the ratio is - under normal circumstances - most stable. A (in particular rapid) change in the ratio may be caused by hemodynamic instability (e.g. during surgery or in ICU), so that altering the practitioner about such change is beneficial, allowing for suitable measures to be taken.

In a second aspect of the present invention a processing unit for a system for determining a representation of cardiac output in a patient is presented, comprising an interface for receiving ultrasound signals received by a transducer unit positioned in the Superior Vena Cava or the Inferior Vena Cava of the patient, wherein the ultrasound signals are received at a first and a second timing in relation to a transmission of ultrasound signals, and a processor, which is arranged to process the received ultrasound signals received, so determine a first and a second velocity of blood, the first velocity of blood being that of blood in the Superior Vena Cava and the second velocity of blood being that of blood in the Inferior Vena Cava, wherein the processor is further arranged to determine the representation of cardiac output from the first and the second velocity.

The present invention allows also for a separate provision of the processing of the information carried by the ultrasound signals, while it may be noted that this does not necessarily also include the control of the generation of the signals.

In a preferred embodiment, the processor of the processing unit is further arranged to control the transducer unit, wherein the controlling includes controlling of the first and a second timing of receiving ultrasound signals in relation to a transmission of ultrasound signals.

In a further aspect of the present invention a software product is presented for determining a representation of cardiac output in a patient.

It shall be understood that the system, the processing unit, and the computer program or software product have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a schematic illustration of a setup in accordance with an embodiment of the invention,
Fig. 2 shows schematically a system in accordance with another embodiment of the invention, and
Fig. 3 shows a schematic flow diagram for illustrating a method according to an embodiment of the invention.

### DETAILED DESCRIPTION OF EMBODIMENTS

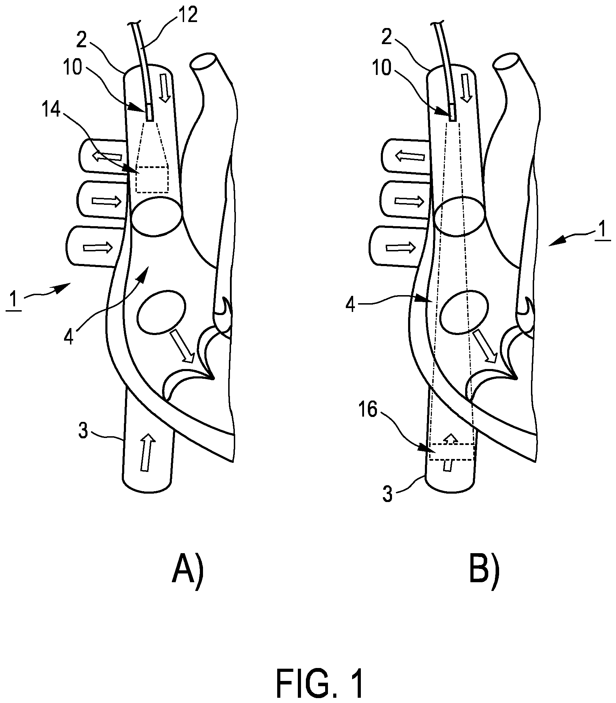

Fig. 1 shows a schematic illustration of a setup in accordance with an embodiment of the invention. Fig. 1 schematically illustrates a part of a heart 1, including the Superior Vena Cava 2 and the Inferior Vena Cava 3, both leading to the Right Atrium 4.

The illustrated setup according to the present invention provides a method and system to monitor cardiac output using a single device inserted into a Central Venous Catheter (CVC) or Peripheral Inserted Central Catheters (PICC) line. In the present preferred embodiment, the device contains an elongated body that fits the lumen of a Central Venous Catheter or Peripheral Inserted Central Catheters line and has an ultrasound transducer at the tip. By operating the device such that the velocity in both the Superior Vena Cava and Inferior Vena Cava can be sampled continuously the CO is monitored.

In an exemplary embodiment, the main elements include an elongated body 10 with an ultrasound transducer at the tip that fits the lumen of a CVC 12 or PICC, a console (not shown in Fig. 1) with a processing algorithm that transmits, receives and processes the ultrasound data into a map of velocities in the Superior Vena Cava 2, that transmits, receives and processes the ultrasound data into a map of velocities in the Inferior Vena Cava 3, and that from both velocity maps determine a measure for the total venous return and thereby CO.

In Fig. 1, a setup according to an embodiment of the invention is schematically depicted. An elongated body 10, with an ultrasound transducer, is inserted into the lumen of a CVC 12 (or PICC) with the US transducer just exiting the CVC. The US transducer can be a single element or an array. It can consist of piezo, single crystal, CMUT or PMUT elements. An ultrasound pulse is transmitted from the transducer and after time Δt the transducer receives the backscattered US waves. By analysis of the received ultrasound signals, the blood velocity can be deduced as in standard ultrasound pulsed Doppler measurements. From the time between transmission and reception of the backscattered ultrasound waves, the distance between the transducer tip and the scattering objects can be deduced. It is, therefore, possible to measure flow velocities from different depths from one series of ultrasound acquisitions. Since the Superior Vena Cava 2 and Inferior Vena Cava 3 are typically aligned, the transducer device in the CVC should also be aligned with both vessels: ideally, the transducer is positioned such that the Superior Vena Cava 2 and Inferior Vena Cava 3 are along the transducer axis. This enables sampling of the blood velocity in both Superior Vena Cava 2 and Inferior Vena Cava 3 without beam steering. The distance between the two vessels is typically 10 cm and the maximum blood velocity in the order of 50 cm/s.

For example, a single-element transducer with an aperture diameter of 1 mm and a center frequency of 1 MHz can be operated to receive signals from up to 15 cm away from the transducer by choosing the pulse repetition frequency (PRF) around 5 kHz. At this PRF, velocities up to 1.4 m/s can be measured without aliasing. This exemplary calculation is meant to show feasibility of the previously stated depth and velocity requirements and, therefore, only serves as an example.

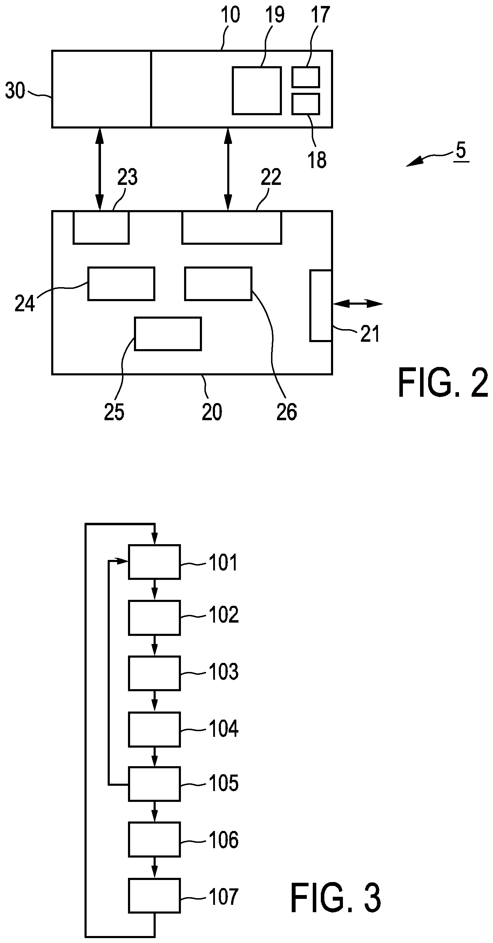

The measurements procedure includes by way of example (as shown in the flow diagram of Fig. 3):
The transducer transmits an ultrasound wave at t=t₀ (Step 101 in Fig. 3).

The transducer goes into receive mode and detects the US-waves that are reflected (Step 102 in Fig. 3).

At time t = t₀ + Δt₁ the signal from the sample area 14 in the Superior Vena Cava is received (left side of Fig. 1, i.e. Fig. 1A) and the blood velocity in the Superior Vena Cava is determined (Step 103 in Fig. 3). Assuming, for example, the region of interest in the Superior Vena Cava to be at 2 cm from the transducer, it can be calculated that Δt₁ = 26 µs. At time t = t₀ + Δt₂ the signal from the sample area 16 in the Inferior Vena Cava is received (right side of Fig. 1, i.e. Fig. 1B) and the blood velocity in the Inferior Vena Cava is determined (Step 104 in Fig. 3).

At time t1 = t₀ + Δt_{PRF}, go back to step 1 until N ultrasound waves are transmitted and their backscattered signals received (Step 105 in Fig. 3).

After receiving the backscattered signals from an ensemble (1...n...N) of ultrasound transmissions, estimate the flow velocity in both the Superior Vena Cava and Inferior Vena Cava by analysis of the received signals at times t₀ + n Δt_{PRF} + Δt₁ and t₀ + n Δt_{PRF} + Δt₂, by a pulsed Doppler velocity estimation algorithm (Step 106 in Fig. 3). This may be based on an autocorrelation as described in "Real-Time Two-Dimensional Blood Flow Imaging Using an Autocorrelation Technique" by Kasai et al. (IEEE Transactions on Sonics and Ultrasonics, Vol. 32(3), pages 458 to 464, 1985) or on a Fourier analysis.

Combine the velocity values in the Inferior Vena Cava and Superior Vena Cava to calculate a value representing the cardiac output by averaging over one or more heart cycles (Step 107 in Fig. 3). This may be performed by addition of the flow velocity in the Superior Vena Cava and the Inferior Vena Cava, where it has to be taken into account that, in the configuration of Fig. 1, the velocities will have a different sign, such that the total velocity is the difference between the measured velocity in the Superior Vena Cava and that measured in the Inferior Vena Cava.

The steps can be repeated to enable continuous monitoring of the cardiac output.

Δt₁ and Δt₂ are directly related to the distance from the transducer: Δt₁ = 2 z₁/c and Δt₁ = 2 z₂/c, where z₁ is the distance between the transducer and a region of interest in the Superior Vena Cava, z₂ is the distance between the transducer and a region of interest in the Inferior Vena Cava, and c is the propagation velocity of ultrasound in blood, which is 1540 m/s.

Δt₁ and Δt₂ can be chosen by estimating z₁ and z₂ based on imaging of the Superior Vena Cava and Inferior Vena Cava anatomy of the patient. Alternatively, they can be determined automatically based on the measured velocity profile, since the blood velocity in the Inferior Vena Cava has a reversed direction and can be easily distinguished from the velocity in the Superior Vena Cava and right atrium.

In a modification or variation of the above embodiment the ultrasound transducer is directly mounted onto the CVC or PICC, thus forming a fully integrated device.

In a modification or variation of the above embodiments the center frequency and pulse length of the US transducer is optimized to sample in the Superior Vena Cava and Inferior Vena Cava respectively. This means that the transmit signal for the two parts of the measurement are different. A possible flow of steps in this regard may include transmitting or sending in a first way for sampling in one of the Superior Vena Cava and the Inferior Vena Cava, changing to a receive mode, and receiving the pulse, transmitting or sending in second way for sampling in the other one of Superior Vena Cava and the Inferior Vena Cava, also changing to the receive mode and receiving the respective pulse, while this flow might be repeated several times, while the exact separation of the steps may not be mandatory.

In a modification or variation of the above embodiments the part of the elongate body, PICC or CVC that contains the ultrasound transducer can be steered toward the optimal position. Possible materials that enable this are: pull-wires, shape memory alloys and electro-active polymers.

In a modification or variation of the above embodiments, changes in the ratio of blood velocity in the Superior Vena Cava and Inferior Vena Cava are used to indicate hemodynamic instability in order to provide further relevant information.

In a modification or variation of the above embodiments, Doppler ultrasound and imaging ultrasound modes are used intermittently. From the ultrasound images the Superior Vena Cava and Inferior Vena Cava can be automatically segmented, allowing for automatic optimization of the sampling windows for blood flow. Also, the diameter of the Superior Vena Cava and Inferior Vena Cava can be automatically determined. In this way, data on the blood flow in the Superior Vena Cava and Inferior Vena Cava is combined with diameter of the Superior Vena Cava and Inferior Vena Cava, for a more accurate calculation of the blood flow into the right atrium.

In a modification or variation of the above embodiments, the diameters of the Superior Vena Cava or Inferior Vena Cava are measured simultaneously with the velocity in order to allow fully quantitative cardiac output measurements. This measurement could be integrated into the device by means of an IVUS transducer. A low IVUS frequency should be adopted to allow sufficient penetration depth for visualization of the vessel wall; rotational or phased-array IVUS configurations can both be used.

In a modification or variation of the above embodiments, the central venous pressure is measured. As the diameter of the vessel varies with the blood pressure, variation in the diameter can be detected via pressure monitoring. The pressure can be measured via a pressure sensor on same device as the ultrasound Doppler sensor or via the lumen of the CVC or PICC with an external pressure transducer.

In a modification or variation of the above embodiments, two distinct transducers elements are mounted on the catheter/guidewire. One transducer is optimized for blood velocity measurements in the Superior Vena Cava the other for the Inferior Vena Cava. Although this is a more complex device, it has the benefit that each transducer can be optimized for the distance at which flow velocity should be measured, and an optimal signal-to-noise ratio can be achieved for both the Superior Vena Cava and Inferior Vena Cava flow velocity measurements. For example, acoustic lenses can be adopted to focus the ultrasound beams at the optimal distances, e.g. 2 cm for Superior Vena Cava and 12 cm for Inferior Vena Cava. Additionally, the center frequency and bandwidth of the transducers can be tuned towards this target distance. Furthermore, if the transducers have no overlap in their frequency band, both elements can be used simultaneously without introducing any crosstalk between the two separate measurements.

Fig. 2 shows schematically a system in accordance with another embodiment of the invention.

The system 5 includes a transducer unit 10, a processor 20 and a pressure sensor 30.

The processor 20 includes an interface 21, a transducer interface 22, a pressure interface 23, a control unit 24, an imaging unit 25 and a processing unit 26.

The interface 21 is provided for receiving input from outside the system 5, e.g. information on the distance from the transducer unit 10 to the sample areas respectively (see Fig. 1) and for providing information to the outside of the system 5, e.g. information on the cardiac output.

The transducer interface 22 allows for a flow of signals between the processor 20 and the transducer unit 10.

The pressure interface 23 is provided to receive pressure information from the pressure sensor 30.

The control unit 24 is coupled to the transducer interface 22 and thus to the transducer unit 10, so to control the operation of the transducer unit 10.

In parallel to the control unit 24, also the imaging unit 25 is coupled, via the transducer interface 22 to the transducer 10, so to operate the transducer unit 10 in an imaging mode and to receive the signal information. The imaging unit 25 processes the signal information received from the transducer unit 10 and provides information to the control unit 24 (e.g. information on the anatomical scale, supplementing or overriding the information provided via the interface 21) and to the processing unit 26 (e.g. information as to the size of the blood vessels).

The processing unit 26 receives, via the transducer interface 22 and the pressure interface 23, ultrasound signals and pressure information, and obtains information therefrom indicative of the cardiac output. Such information is outputted via the interface 21.

The transducer unit 10 includes two transducer elements 17, 18, wherein one of those elements is provided and optimized for determining the blood flow velocity in the Superior Vena Cava, while the other one is provided and optimized for determining the blood flow velocity in the Inferior Vena Cava (see Fig. 1). It is noted that in other embodiments of the invention there might be just one transducer element, while it is also contemplated that there might be more than two transducer elements.

Further, the transducer unit 10 includes a steering element 19, which is arranged for changing the operation direction of the transducer unit 10, more specifically the operation direction of the transducer elements 17, 18, either commonly or independently.

To a certain degree irrespective of the orientation of the CVC (or PICC) (see Fig. 1), due to the steering element 19, the ultrasound transmission and reception by means of the transducer elements 17, 18 can be directed to the respective sample areas (see Fig. 1).

The pressure sensor 30 provides pressure information to the processor 20.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, device or other unit may fulfill the functions of several items recited in the claims. The mere fact that certain measures are recited in mutually different dependent claims does not indicate that a combination of these measures cannot be used to advantage.

Operations like controlling, processing, determining, and calculating can be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A system (5) for determining a representation of cardiac output in a patient, comprising:
a transducer unit (10) configured for transmitting to and receiving ultrasound signals from the Superior Vena Cava (2) and the Inferior Vena Cava (3) of the patient, and
a processor (20) configured
to control the transducer unit (10), wherein the controlling includes controlling of a first and a second timing of receiving ultrasound signals in relation to a transmission of ultrasound signals, and
to process the ultrasound signals received at the first and the second timing, so as to determine a first and a second velocity of blood, the first velocity of blood being that of blood in the Superior Vena Cava (2) and the second velocity of blood being that of blood in the Inferior Vena Cava (3),
wherein the processor (20) is further configured to be arranged to determine the representation of cardiac output from the first and the second velocity.

2. The system (5) for determining the representation of cardiac output according to claim 1, wherein the processor (20) is further configured to be arranged
to determine the first and second timing based on distance information inputted to the system (5), the distance information indicating a distance between the transducer unit (10) and a region of interest (14) in the Superior Vena Cava (2) and between the transducer unit (10) and a region of interest (16) in the Inferior Vena Cava (3), and/or
to determine the first and second timing based on a velocity profile along a line of sight of the transducer unit (10).

3. The system (5) for determining the representation of cardiac output according to claim 1,
wherein the processor (20) is further configured to be arranged to control the transducer unit (10) in an imaging mode, and
wherein the processor (20) is further configured to be arranged
to derive anatomical information from an ultrasound image and to determine the first and second timing based on the derived anatomical information; and/or
to derive anatomical information from an ultrasound image, wherein the anatomical information includes a diameter of the Superior Vena Cava (2) and/or the Inferior Vena Cava (3), wherein the diameter is used in determining the representation of the cardiac output.

4. The system (5) for determining the representation of cardiac output according to claim 3,
wherein the transducer unit (10) includes an intravascular ultrasound transducer.

5. The system (5) for determining the representation of cardiac output according to claim 1,
wherein the processor (20) is configured to be arranged to control the transducer unit (10) so to provide a series of transmission and receptions, and
wherein the processor (20) is configured to be arranged to determine the first and the second velocity from an ensemble of received ultrasound signals corresponding to the series of receptions.

6. The system (5) for determining the representation of cardiac output according to claim 5,
wherein the representation of cardiac output is determined by averaging over one or more heart cycles.

7. The system (5) for determining the representation of cardiac output according to claim 1,
wherein the processor (20) is configured to be arranged to control the transducer unit (10) such that a first and a second ultrasound signal is transmitted, wherein the first timing of receiving is in relation to the transmission of the first ultrasound signal and the second timing of receiving is in relation to the transmission of the second ultrasound signal.

8. The system (5) for determining the representation of cardiac output according to claim 1,
wherein the transducer unit (10) includes a first transducer element (17) and a second transducer element (18), and
wherein the processor is configured to be arranged to independently control the first and the second transducer element (17, 18), wherein the first timing of receiving is in relation to a transmission of an ultrasound signal by the first transducer element (17) and the second timing of receiving is in relation to a transmission of an ultrasound signal by the second transducer element (18).

9. The system (5) for determining the representation of cardiac output according to claim 1,
wherein the transducer unit (10) is configured to be provided such that the orientation of the transducer unit (10), a transmitted beam and/or a received beam is adjustable and
wherein the processor (20) is further configured to be arranged to adjust an orientation of the transducer unit (10), the transmitted beam and/or the received beam, so to provide a first orientation at the first timing of receiving and a second orientation at the second timing of receiving.

10. The system (5) for determining the representation of cardiac output according to claim 1,
wherein the system (5) further comprises a pressure sensor (30) configured detecting a pressure in the Superior Vena Cava (2) or the Inferior Vena Cava (3) of the patient,
wherein the processor (20) is configured to be arranged to use the detected pressure in the determination of the representation of cardiac output.

11. The system (5) for determining the representation of cardiac output according to claim 1,
wherein the processor (20) is further configured to be arranged to monitor a change in a ratio between the first and the second velocity over time and to provide a notification in case the change exceeds a predetermined threshold.

12. A processor (20) for a system for determining a representation of cardiac output in a patient, comprising:
an interface (22) configured for receiving ultrasound signals received by a transducer unit (10) positioned in the Superior Vena Cava (2) and the Inferior Vena Cava (3) of the patient,
wherein the ultrasound signals are received at a first and a second timing in relation to a transmission of ultrasound signals, and
a processing unit (26), which is configured to be arranged to process the received ultrasound signals received, so as to determine a first and a second velocity of blood, the first velocity of blood being that of blood in the Superior Vena Cava (2) and the second velocity of blood being that of blood in the Inferior Vena Cava (3),
wherein the processing unit (26) is further configured to be arranged to determine the representation of cardiac output from the first and the second velocity.

13. The processor (20) according to claim 12,
wherein the processing unit (26) is further configured to be arranged to control the transducer unit (10),
wherein the controlling includes controlling of the first and a second timing of receiving ultrasound signals in relation to a transmission of ultrasound signals.

14. A software product for determining a representation of cardiac output in a patient, the software product comprising program code means for causing a processor (20) to carry out, when run on the system (5) according to claim 1, the steps:
causing a transducer unit (10) positioned in the Superior Vena Cava (2) and the Inferior Vena Cava (3) of the patient to transmit (101) and to receive (102, 103, 104) ultrasound signals, wherein the receiving of ultrasound signals is provided at a first and a second timing in relation to a transmission of ultrasound signals,
processing (106) the ultrasound signals received at the first and the second timing, so as to determine a first and a second velocity of blood, the first velocity of blood being that of blood in the Superior Vena Cava (2) and the second velocity of blood being that of blood in the Inferior Vena Cava (3), and
determining (107) a representation of a cardiac output from the first and the second velocity.

## Patentansprüche

1. System (5) zur Bestimmung einer Darstellung der Herzleistung bei einem Patienten, umfassend:
eine Wandlereinheit (10), die konfiguriert ist, um Ultraschallsignale von der oberen Vena Cava (2) oder der unteren Vena Cava (3) des Patienten zu senden und zu empfangen, und
einen Prozessor (20), der konfiguriert ist, um die Wandlereinheit (10) zu steuern, wobei das Steuern das Steuern eines ersten und eines zweiten Zeitpunkts des Empfangs von Ultraschallsignalen in Bezug auf eine Übertragung von Ultraschallsignalen einschließt, und
um die Ultraschallsignale zu verarbeiten, die beim ersten und zweiten Zeitpunkt empfangen werden, um eine erste und eine zweite Geschwindigkeit des Blutes zu bestimmen, wobei die erste Geschwindigkeit des Blutes die des Blutes in der oberen Vena Cava (2) und die zweite Geschwindigkeit des Blutes die des Blutes in der unteren Vena Cava (3) ist,
wobei der Prozessor (20) weiter konfiguriert ist, um angeordnet zu werden, um die Darstellung der Herzleistung aus der ersten und zweiten Geschwindigkeit zu bestimmen.

2. System (5) zur Bestimmung der Darstellung der Herzleistung nach Anspruch 1, wobei der Prozessor (20) weiter konfiguriert ist, um angeordnet zu werden,
um den ersten und zweiten Zeitpunkt basierend auf den in das System (5) eingegebenen Abstandsinformationen zu bestimmen, wobei die Abstandsinformationen einen Abstand zwischen der Wandlereinheit (10) und einem Bereich von Interesse (14) in der oberen Vena Cava (2) und zwischen der Wandlereinheit (10) und einem Bereich von Interesse (16) in der unteren Vena Cava (3) angeben, und/oder
um den ersten und zweiten Zeitpunkt basierend auf einem Geschwindigkeitsprofil entlang einer Sichtlinie der Wandlereinheit (10) zu bestimmen.

3. System (5) zur Bestimmung der Darstellung der Herzleistung nach Anspruch 1,
wobei der Prozessor (20) weiter konfiguriert ist, um angeordnet zu werden, um die Wandlereinheit (10) in einem Abbildungsmodus zu steuern, und
wobei der Prozessor (20) weiter konfiguriert ist, um angeordnet zu werden, um anatomische Informationen aus einem Ultraschallbild abzuleiten und den ersten und zweiten Zeitpunkt basierend auf den abgeleiteten anatomischen Informationen zu bestimmen; und/oder
um anatomische Informationen aus einem Ultraschallbild abzuleiten, wobei die anatomischen Informationen einen Durchmesser der oberen Vena Cava (2) und/oder der unteren Vena Cava (3) einschließen, wobei der Durchmesser zur Bestimmung der Darstellung der Herzleistung verwendet wird.

4. System (5) zur Bestimmung der Darstellung der Herzleistung nach Anspruch 3,
wobei die Wandlereinheit (10) einen intravaskulären Ultraschallwandler einschließt.

5. System (5) zur Bestimmung der Darstellung der Herzleistung nach Anspruch 1,
wobei der Prozessor (20) konfiguriert ist, um angeordnet zu werden, um die Wandlereinheit (10) zu steuern, so dass eine Reihe von Übertragungs- und Empfangsvorgängen bereitgestellt wird, und
wobei der Prozessor (20) konfiguriert ist, um angeordnet zu werden, um die erste und die zweite Geschwindigkeit aus einer Gesamtheit von empfangenen Ultraschallsignalen zu bestimmen, die der Reihe von Empfangsvorgängen entsprechen.

6. System (5) zur Bestimmung der Darstellung der Herzleistung nach Anspruch 5,
wobei die Darstellung der Herzleistung durch Mittelung über einen oder mehrere Herzzyklen bestimmt wird.

7. System (5) zur Bestimmung der Darstellung der Herzleistung nach Anspruch 1,
wobei der Prozessor (20) konfiguriert ist, um angeordnet zu werden, um die Wandlereinheit (10) so zu steuern, dass ein erstes und ein zweites Ultraschallsignal übertragen wird, wobei der erste Zeitpunkt des Empfangs in Bezug auf die Übertragung des ersten Ultraschallsignals und der zweite Zeitpunkt des Empfangs in Bezug auf die Übertragung des zweiten Ultraschallsignals steht.

8. System (5) zur Bestimmung der Darstellung der Herzleistung nach Anspruch 1,
wobei die Wandlereinheit (10) ein erstes Wandlerelement (17) und ein zweites Wandlerelement (18) einschließt, und
wobei der Prozessor konfiguriert ist, um angeordnet zu werden, um das erste und das zweite Wandlerelement (17, 18) unabhängig zu steuern, wobei der erste Zeitpunkt des Empfangs in Bezug auf eine Übertragung eines Ultraschallsignals durch das erste Wandlerelement (17) und der zweite Zeitpunkt des Empfangs in Bezug auf eine Übertragung eines Ultraschallsignals durch das zweite Wandlerelement (18) steht.

9. System (5) zur Bestimmung der Darstellung der Herzleistung nach Anspruch 1,
wobei die Wandlereinheit (10) konfiguriert ist, um so bereitgestellt zu werden, dass die Ausrichtung der Wandlereinheit (10), eines Sendestrahls und/oder eines Empfangsstrahls einstellbar ist und
wobei der Prozessor (20) weiter konfiguriert ist, um angeordnet zu werden, um eine Ausrichtung der Wandlereinheit (10), des Sendestrahls und/oder des Empfangsstrahls einzustellen, so dass eine erste Ausrichtung zum ersten Zeitpunkt des Empfangs und eine zweite Ausrichtung zum zweiten Zeitpunkt des Empfangs bereitgestellt wird.

10. System (5) zur Bestimmung der Darstellung der Herzleistung nach Anspruch 1,
wobei das System (5) weiter einen Drucksensor (30) umfasst, der konfiguriert ist, um einen Druck in der oberen Vena Cava (2) oder der unteren Vena Cava (3) des Patienten zu erfassen,
wobei der Prozessor (20) konfiguriert ist, um angeordnet zu werden, um den erfassten Druck bei der Bestimmung der Darstellung der Herzleistung zu verwenden.

11. System (5) zur Bestimmung der Darstellung der Herzleistung nach Anspruch 1,
wobei der Prozessor (20) weiter konfiguriert ist, um angeordnet zu werden, um eine Änderung in einem Verhältnis zwischen der ersten und der zweiten Geschwindigkeit über die Zeit zu überwachen und eine Meldung bereitzustellen, falls die Änderung einen vorbestimmten Schwellenwert überschreitet.

12. Prozessor (20) für ein System zur Darstellung der Herzleistung bei einem Patienten, umfassend:
eine Schnittstelle (22), die zum Empfangen von Ultraschallsignalen konfiguriert ist, die von einer Wandlereinheit (10) empfangen werden, die in der oberen Vena Cava (2) und der unteren Vena Cava (3) des Patienten angeordnet ist,
wobei die Ultraschallsignale zu einem ersten und einem zweiten Zeitpunkt in Bezug auf eine Übertragung von Ultraschallsignalen empfangen werden, und
eine Verarbeitungseinheit (26), die konfiguriert ist, um angeordnet zu werden, um die empfangenen Ultraschallsignale zu verarbeiten, so dass eine erste und eine zweite Geschwindigkeit des Blutes bestimmt wird, wobei die erste Geschwindigkeit des Blutes die des Blutes in der oberen Vena Cava (2) und die zweite Geschwindigkeit des Blutes die des Blutes in der unteren Vena Cava (3) ist,
wobei die Verarbeitungseinheit (26) weiter konfiguriert ist, um angeordnet zu werden, um die Darstellung der Herzleistung aus der ersten und zweiten Geschwindigkeit zu bestimmen.

13. Prozessor (20) nach Anspruch 12,
wobei die Verarbeitungseinheit (26) weiter konfiguriert ist, um angeordnet zu werden, um die Wandlereinheit (10) zu steuern,
wobei das Steuern das Steuern des ersten und eines zweiten Zeitpunkts des Empfangens von Ultraschallsignalen in Bezug auf eine Übertragung von Ultraschallsignalen einschließt.

14. Softwareprodukt zur Bestimmung einer Darstellung der Herzleistung bei einem Patienten, wobei das Softwareprodukt Programmcodemittel umfasst, um einen Prozessor (20) zu veranlassen, bei Ausführung auf dem System (5) nach Anspruch 1 die folgenden Schritte auszuführen:
Veranlassen einer Wandlereinheit (10), die in der oberen Vena Cava (2) und der unteren Vena Cava (3) des Patienten angeordnet ist, Ultraschallsignale zu senden (101) und zu empfangen (102, 103, 104), wobei das Empfangen von Ultraschallsignalen zu einem ersten und einem zweiten Zeitpunkt in Bezug auf eine Übertragung von Ultraschallsignalen bereitgestellt wird,
Verarbeiten (106) der Ultraschallsignale, die beim ersten und zweiten Zeitpunkt empfangen werden, um eine erste und eine zweite Geschwindigkeit des Blutes zu bestimmen, wobei die erste Geschwindigkeit des Blutes die des Blutes in der oberen Vena Cava (2) und die zweite Geschwindigkeit des Blutes die des Blutes in der unteren Vena Cava (3) ist, und
Bestimmen (107) einer Darstellung einer Herzleistung aus der ersten und zweiten Geschwindigkeit.

## Revendications

1. Système (5) de détermination d'une représentation du débit cardiaque chez un patient, comprenant :
une unité de transducteur (10) configurée pour transmettre et recevoir des signaux ultrasonores de la veine cave supérieure (2) et de la veine cave inférieure (3) du patient, et
un processeur (20) configuré
pour commander l'unité de transducteur (10), dans lequel la commande inclut la commande d'un premier et d'un second moment de réception de signaux ultrasonores en rapport avec une transmission de signaux ultrasonores, et
pour traiter les signaux ultrasonores reçus au premier et au second moment, de façon à déterminer une première et une seconde vitesse du sang, la première vitesse du sang étant celle du sang dans la veine cave supérieure (2) et la seconde vitesse du sang étant celle du sang dans la veine cave inférieure (3),
dans lequel le processeur (20) est en outre configuré pour être agencé pour déterminer la représentation du débit cardiaque de la première et de la seconde vitesse.

2. Système (5) de détermination de la représentation du débit cardiaque selon la revendication 1, dans lequel le processeur (20) est en outre configuré pour être agencé
pour déterminer les premier et second moments sur la base des informations de distance entrées dans le système (5), les informations de distance indiquant une distance entre l'unité de transducteur (10) et une région d'intérêt (14) dans la veine cave supérieure (2) et entre l'unité de transducteur (10) et une région d'intérêt (16) dans la veine cave inférieure (3), et/ou
- pour déterminer les premier et second moments sur la base d'un profil de vitesse le long d'une ligne de vue de l'unité de transducteur (10).

3. Système (5) de détermination de la représentation du débit cardiaque selon la revendication 1,
dans lequel le processeur (20) est en outre configuré pour être agencé pour commander l'unité de transducteur (10) en mode imagerie, et
dans lequel le processeur (20) est en outre configuré pour être agencé
pour dériver des informations anatomiques d'une image ultrasonore et pour déterminer les premier et second moments sur la base des informations anatomiques dérivées ; et/ou
pour dériver des informations anatomiques d'une image ultrasonore, dans lequel les informations anatomique incluent un diamètre de la veine cave supérieure (2) et/ou de la veine cave inférieure (3), dans lequel le diamètre est utilisé pour déterminer la représentation du débit cardiaque.

4. Système (5) de détermination de la représentation du débit cardiaque selon la revendication 3,
dans lequel l'unité de transducteur (10) inclut un transducteur ultrasonore intravasculaire.

5. Système (5) de détermination de la représentation du débit cardiaque selon la revendication 1,
dans lequel le processeur (20) est configuré pour être agencé pour commander l'unité de transducteur (10) de façon à fournir une série de transmission et de réceptions, et
dans lequel le processeur (20) est configuré pour être agencé pour déterminer la première et la seconde vitesse d'un ensemble de signaux ultrasonores reçus correspondant à la série de réceptions.

6. Système (5) de détermination de la représentation du débit cardiaque selon la revendication 5,
dans lequel la représentation du débit cardiaque est déterminée en faisant une moyenne sur un ou plusieurs cycles cardiaques.

7. Système (5) de détermination de la représentation du débit cardiaque selon la revendication 1,
dans lequel le processeur (20) est configuré pour être agencé pour commander l'unité de transducteur (10) de façon à ce qu'un premier et un second signal ultrasonore soient transmis, dans lequel le premier moment de réception est en rapport avec la transmission du premier signal ultrasonore et le second moment de réception est en rapport avec la transmission du second signal ultrasonore.

8. Système (5) de détermination de la représentation du débit cardiaque selon la revendication 1,
dans lequel l'unité de transducteur (10) inclut un premier élément de transducteur (17) et un second élément de transducteur (18), et
dans lequel le processeur est configuré pour être agencé pour commander indépendamment le premier et le second élément de transducteur (17, 18), dans lequel le premier moment de réception est en rapport avec une transmission d'un signal ultrasonore par le premier élément de transducteur (17) et le second moment de réception est en rapport avec une transmission d'un signal ultrasonore par le second élément de transducteur (18).

9. Système (5) de détermination de la représentation du débit cardiaque selon la revendication 1,
dans lequel l'unité de transducteur (10) est configurée pour être fournie de manière à ce que l'orientation de l'unité de transducteur (10), un faisceau transmis et/ou un faisceau reçu soient ajustables et
dans lequel le processeur (20) est en outre configuré pour être agencé pour ajuster une orientation de l'unité de transducteur (10), du faisceau transmis et/ou du faisceau reçu, pour fournir une première orientation au premier moment de réception et une seconde orientation au second moment de réception.

10. Système (5) de détermination de la représentation du débit cardiaque selon la revendication 1,
dans lequel le système (5) comprend en outre un capteur de pression (30) configuré pour détecter une pression dans la veine cave supérieure (2) ou la veine cave inférieure (3) du patient,
dans lequel le processeur (20) est configuré pour être agencé pour utiliser la pression détectée dans la détermination de la représentation du débit cardiaque.

11. Système (5) de détermination de la représentation du débit cardiaque selon la revendication 1,
dans lequel le processeur (20) est en outre configuré pour être agencé pour surveiller une modification d'un rapport entre la première et la seconde vitesse dans le temps pour fournir une notification dans le cas où la modification excède un seuil prédéterminé.

12. Processeur (20) pour un système de détermination d'une représentation du débit cardiaque chez un patient, comprenant :
une interface (22) configurée pour recevoir des signaux ultrasonores reçus par une unité de transducteur (10) positionnée dans la veine cave supérieure (2) ou la veine cave inférieure (3) du patient,
dans lequel les signaux ultrasonores sont reçus à un premier et un second moment en rapport avec une transmission de signaux ultrasonores, et
une unité de traitement (26), qui est configurée pour être agencée pour traiter les signaux ultrasonores reçus, de façon à déterminer une première et une seconde vitesse du sang, la première vitesse du sang étant celle du sang dans la veine cave supérieure (2) et la seconde vitesse du sang étant celle du sang dans la veine cave inférieure (3),
dans lequel l'unité de traitement (26) est en outre configurée pour être agencée pour déterminer la représentation du débit cardiaque à partir de la première et de la seconde vitesse.

13. Processeur (20) selon la revendication 12,
dans lequel l'unité de traitement (26) est en outre configurée pour être agencée pour commander l'unité de transducteur (10),
dans lequel la commande inclut la commande du premier et d'un second moment de réception de signaux ultrasonores en rapport avec une transmission de signaux ultrasonores.

14. Produit de logiciel pour déterminer une représentation du débit cardiaque chez un patient, le produit de logiciel comprenant un moyen de code de programme pour amener un processeur (20) à réaliser, lorsqu'il est utilisé sur le système (5) selon la revendication 1, les étapes suivantes :
amener une unité de transducteur (10) positionnée dans la veine cave supérieure (2) et la veine cave inférieure (3) du patient à transmettre (101) et recevoir (102, 103, 104) des signaux ultrasonores, dans lequel la réception des signaux ultrasonores est prévue à un premier et un second moment en rapport avec une transmission de signaux ultrasonores,
traiter (106) les signaux ultrasonores reçus au premier et au second moment, de façon à déterminer une première et une seconde vitesse du sang, la première vitesse du sang étant celle du sang dans la veine cave supérieure (2) et la seconde vitesse du sang étant celle du sang dans la veine cave inférieure (3), et
déterminer (107) une représentation du débit cardiaque à partir de la première et de la seconde vitesse.
